# NEW EUROPEAN PATENT SPECIFICATION

(11) **EP 3 068 403 B2**
(45) Date of publication and mention of the opposition decision: **03.12.2025**
(45) Mention of the grant of the patent: 17.06.2020
(21) Application number: 14805768.0
(22) Date of filing: 14.11.2014
(51) Int. Cl.: A61K 31/573, A61K 9/00, A61K 47/34, A61P 27/02

(54) **METHODS OF TREATMENT OF OCULAR CONDITIONS WITH A SUSTAINED DRUG DELIVERY IMPLANT**
VERFAHREN ZUR BEHANDLUNG VON OKULAREN KRANKHEITEN MITTES EINEM IMPLANTAT MIT VERZÖGERTER WIRKSTOFFFREISETZUNG
METHODES POUR LE TRAITEMENT DE TROUBLES OCULAIRES AVEC UN IMPLANT À ADMINISTRATION DE MÉDICAMENT PROLONGÉE

(30) Priority: 15.11.2013 US 201361904887 P
(43) Date of publication of application: 21.09.2016
(60) Divisional application: 20173457.1
(73) Proprietor: ALLERGAN, INC., Irvine, CA 92612 (US)
(72) Inventor: SHIAH, Jane-Guo, Irvine, California 92620 (US); BHAGAT, Rahul, Irvine, California 92620 (US); BLANDA, Wendy M., Tustin, California 92780 (US); NIVAGGIOLI, Thierry, Atherton, California 94027 (US); PENG, Lin, South San Francisco, California 94080 (US); CHOU, David, Palo Alto, California 94306 (US); WEBER, David A., Danville, CA 94506-6004 (US)
(74) Representative: Hoffmann Eitle
(86) International application number: PCT/US2014/065804
(87) International publication number: WO 2015/073895

(56) References cited:
- US-A1- 2005 244 469
- US-B2- 8 034 366
- SALMAN, A.G.: "Long-term effect of intravitreal dexamethasone implant (Ozurdex) in the treatment of resistant diabetic macular edema", JOURNAL OF EGYPTIAN OPHTHALMOLOGICAL SOCIETY, vol. 106, no. 3, 2013, pages 194 - 198, XP055795706
- "History of Changes for Study: NCT00168337", 30 May 2013 (2013-05-30), XP055795698, Retrieved from the Internet <URL:https://clinicaltrials.gov/ct2/history/NCT00168337?V_13=View#StudyPageTop>
- "History of Changes for Study: NCT00168389", 21 June 2013 (2013-06-21), XP055795702, Retrieved from the Internet <URL:https://clinicaltrials.gov/ct2/history/NCT00168389?V_13=View#StudyPageTop>
- CROSS-DISCIPLINE TEAM LEADER REVIEW, WILLIAM M BOYD, M.D.: "NDA 22-315; OZURDEX (dexamethasone intravitreal implant)", CENTER FOR DRUG EVALUATION AND RESEARCH, 17 June 2009 (2009-06-17), XP002734614, Retrieved from the Internet <URL:http://www.accessdata.fda.gov/drugsatfda_docs/nda/2009/022315s000_CrossR.pdf> [retrieved on 20150116]
- KUPPERMANN BARUCH D ET AL: "Randomized controlled study of an intravitreous dexamethasone drug delivery system in patients with persistent macular edema.", ARCHIVES OF OPHTHALMOLOGY MAR 2007, vol. 125, no. 3, March 2007 (2007-03-01), pages 309 - 317, XP009181957, ISSN: 0003-9950
- HALLER J A ET AL: "Randomized Controlled Trial of an Intravitreous Dexamethasone Drug Delivery System in Patients With Diabetic Macular Edema", ARCHIVES OF OPHTHALMOLOGY, AMERICAN MEDICAL ASSOCIATION, US, vol. 128, no. 3, March 2010 (2010-03-01), pages 289 - 296, XP008170031, ISSN: 0003-9950
- PACELLA ELENA ET AL: "Preliminary results of an intravitreal dexamethasone implant (Ozurdex(R)) in patients with persistent diabetic macular edema.", CLINICAL OPHTHALMOLOGY (AUCKLAND, N.Z.) 2013, vol. 7, 16 July 2013 (2013-07-16), pages 1423 - 1428, XP055162531, ISSN: 1177-5467, DOI: 10.2147/OPTH.S48364
- HALLER JULIA A ET AL: "Dexamethasone Intravitreal Implant in Patients with Macular Edema Related to Branch or Central Retinal Vein Occlusion", OPHTHALMOLOGY, J. B. LIPPINCOTT CO., PHILADELPHIA, PA, US, vol. 118, no. 12, 10 May 2011 (2011-05-10), pages 2453 - 2460, XP028397162, ISSN: 0161-6420, [retrieved on 20110517], DOI: 10.1016/J.OPHTHA.2011.05.014
- BOYER DAVID S ET AL: "Three-year, randomized, sham-controlled trial of dexamethasone intravitreal implant in patients with diabetic macular edema.", OPHTHALMOLOGY OCT 2014, vol. 121, no. 10, October 2014 (2014-10-01), pages 1904 - 1914, XP055162551, ISSN: 0161-6420, DOI: 10.1016/j.ophtha.2014.04.024

## Description

### BACKGROUND

### Field

The disclosure of the present application generally relates to drug delivery implants, and more specifically, drug delivery implants for use in a method for treating ocular conditions.

### Description of the Related Art

Macular edema ("ME") is an ocular condition that can result in a swelling of the macula. The edema is caused by fluid leaking from retinal blood vessels. Blood leaks out of the weak vessel walls into a very small area of the macula which is rich in cones, the nerve endings that detect color and from which daytime vision depends. Blurring then occurs in the middle or just to the side of the central visual field. Visual loss can progress over a period of months. Retinal blood vessel obstruction, eye inflammation, and age-related macular degeneration have all been associated with macular edema. The macula may also be affected by swelling following cataract extraction. Symptoms of ME include blurred central vision, distorted vision, vision tinted pink and light sensitivity. Causes of ME can include retinal vein occlusion, macular degeneration, diabetic macular leakage, eye inflammation, idiopathic central serous chorioretinopathy, anterior or posterior uveitis, pars planitis, retinitis pigmentosa, radiation retinopathy, posterior vitreous detachment, epiretinal membrane formation, idiopathic juxtafoveal retinal telangiectasia, Nd:YAG capsulotomy or iridotomy. Some patients with ME may have a history of use of topical epinephrine or prostaglandin analogs for glaucoma.

Macular edema involves the breakdown of the inner blood retinal barrier at the level of the capillary endothelium, resulting in abnormal retinal vascular permeability and leakage into the adjacent retinal tissues. The macula becomes thickened due to fluid accumulation resulting in significant disturbances in visual acuity.

Macular edema may occur in diseases causing cumulative injury over many years, such as diabetic retinopathy, or as a result of more acute events, such as central retinal vein occlusion or branch retinal vein occlusion.

Diabetic retinopathy is a frequent microvascular complication of diabetes types 1 and 2 and represents the leading cause of blindness in the world. Diabetes-related central vision loss can arise either from microvascular occlusion (mascular ischemia) or from microvascular leakage due to breakdown of the inner blood-retinal barrier (BRB), leading to thickening or swelling of the macula (macular edema). Diabetic macular edema (DME) affects an estimated 21 million individuals worldwide.

Several treatment options have emerged that offer to improve visual acuity, including intravitreal anti-vascular endothelial growth factor (anti-VEGF) agents and laser photocoagulation. However, these treatment options have some drawbacks and do not work effectively for all patients.

Haller J A et al, Archives of Ophthalmology, vol. 128, no. 3, March 2010, pages 289-296, discloses a randomised controlled trail of an intravitreous dexamethasone drug delivery system in patients with diabetic macular edema.

### SUMMARY

The present invention is directed to a bioerodible implant for use in a method for treating diabetic macular edema, the method comprising injecting said bioerodible implant into the vitreous of a human at a frequency of once every 7 months, 8 months or 9 months, the bioerodible implant comprising a continuous, double extruded rod comprising dexamethasone homogeneously dispersed within a biodegradable polymer matrix; wherein the biodegradable polymer matrix comprises a mixture of poly(D,L-lactide-co-glycolide) (PLGA) having hydrophilic end groups and poly(D,L-lactide-co-glycolide) (PLGA) having hydrophobic end groups; wherein the bioerodible implant is sized for implantation in the vitreous of the human; and wherein the method is therapeutically effective to treat DME; and wherein said bioerodible implant is administered for a period of time of 2 years, 3 years, 4 years, 5 years, 10 years, 15 years, or for the lifetime of said human. Further embodiments of the invention are specified in the claims.

Embodiments disclosed herein which do not fall within the scope of the claims do not form part of the invention.

Disclosed herein are implants and implants for use in the treatment of diabetic macular edema ("DME"). The implant contains dexamethasone.

An ocular condition can include a disease, aliment or condition which affects or involves the eye or one of the parts or regions of the eye. Broadly speaking the eye includes the eyeball and the tissues and fluids which constitute the eyeball, the periocular muscles (such as the oblique and rectus muscles) and the portion of the optic nerve which is within or adjacent to the eyeball. An anterior ocular condition is a disease, ailment or condition which affects or which involves an anterior (i.e. front of the eye) ocular region or site, such as a periocular muscle, an eye lid or an eye ball tissue or fluid which is located anterior to the posterior wall of the lens capsule or ciliary muscles. Thus, an anterior ocular condition primarily affects or involves, the conjunctiva, the cornea, the conjunctiva, the anterior chamber, the iris, the posterior chamber (behind the retina but in front of the posterior wall of the lens capsule), the lens or the lens capsule and blood vessels and nerve which vascularize or innervate an anterior ocular region or site. A posterior ocular condition is a disease, ailment or condition which primarily affects or involves a posterior ocular region or site such as choroid or sclera (in a position posterior to a plane through the posterior wall of the lens capsule), vitreous, vitreous chamber, retina, optic nerve (i.e. the optic disc), and blood vessels and nerves which vascularize or innervate a posterior ocular region or site.

A posterior ocular condition can include a disease, ailment or condition, such as for example, macular degeneration (such as non-exudative age related macular degeneration and exudative age related macular degeneration); choroidal neovascularization; acute macular neuroretinopathy; macular edema (such as cystoid macular edema and diabetic macular edema); Behcet's disease, retinal disorders, diabetic retinopathy (including proliferative diabetic retinopathy); retinal arterial occlusive disease; central retinal vein occlusion; uveitic retinal disease; retinal detachment; ocular trauma which affects a posterior ocular site or location; a posterior ocular condition caused by or influenced by an ocular laser treatment; posterior ocular conditions caused by or influenced by a photodynamic therapy; photocoagulation; radiation retinopathy; epiretinal membrane disorders; branch retinal vein occlusion; anterior ischemic optic neuropathy; non-retinopathy diabetic retinal dysfunction, retinitis pigmentosa and glaucoma.

An anterior ocular condition can include a disease, ailment or condition, such as for example, aphakia; pseudophakia; astigmatism; blepharospasm; cataract; conjunctival diseases; conjunctivitis; corneal diseases; corneal ulcer; dry eye syndromes; eyelid diseases; lacrimal apparatus diseases; lacrimal duct obstruction; myopia; presbyopia; pupil disorders; refractive disorders and strabismus. Glaucoma can also be considered to be an anterior ocular condition because a clinical goal of glaucoma treatment can be to reduce a hypertension of aqueous fluid in the anterior chamber of the eye (i.e. reduce intraocular pressure).

Potent corticosteroids such as dexamethasone suppress inflammation by inhibiting edema, fibrin deposition, capillary leakage and phagocytic migration, all key features of the inflammatory response. Corticosteroids prevent the release of prostaglandins, some of which have been identified as mediators of cystoid macular edema.

By delivering a drug, such as a corticosteroid, directly into the vitreous cavity, blood eye barriers can be circumvented and intraocular therapeutic levels can be achieved with minimal risk of systemic toxicity. This route of administration typically results in a short half-life unless the drug can be delivered using a formulation capable of providing sustained release.

Consequently, a biodegradable implant for delivering a therapeutic agent to an ocular region, such as the vitreous may provide significant medical benefit for patients afflicted with a medical condition of the eye, such as diabetic macular edema.

According to an embodiment a bioerodible implant for use in a method for treating diabetic macular edema includes injecting the bioerodible implant into the vitreous of a human in need thereof at a frequency of once every 7 months, 8 months or 9 months. The bioerodible implant includes a continuous, double extruded rod including dexamethasone homogeneously dispersed within a biodegradable polymer matrix. The biodegradable polymer matrix includes a mixture of poly(D,L-lactide-co-glycolide) (PLGA) having hydrophilic end groups and poly(D,L-lactide-co-glycolide) (PLGA) having hydrophobic end groups. The bioerodible implant is sized for implantation in the vitreous of the human. The method is therapeutically effective to treat DME The active agent is dexamethasone. The bioerodible implant is administered for a period of time of 2 years, 3 years, 4 years, 5 years, 10 years, 15 years, or for the lifetime of said human. In some embodiments, the macular edema is diabetic macular edema. In some embodiments, the dexamethasone is present in the bioerodible implant in an amount of 60% by weight, based on the total weight of the bioerodible implant. In some embodiments, the PLGA having hydrophobic end groups is present in the bioerodible implant in an amount of 10% by weight, based on the total weight of the bioerodible implant. In some embodiments, the PLGA having hydrophilic end groups is present in the bioerodible implant in an amount of 30% by weight, based on the total weight of the bioerodible implant. According to an embodiment, the human has a pseudophakic lens. According to another embodiment, the human has a phakic lens.

### BRIEF DESCRIPTION OF THE FIGURES

These and other features will now be described with reference to the drawings summarized below. These drawings and the associated description are provided to illustrate one or more embodiments.
Figure 1 illustrates a bar graph comparing the proportion of DME patients having a BCVA improvement of greater than or equal to 15 letters in patient groups receiving example embodiment bioerodible implants according to example embodiment methods disclosed herein.
Figure 2 illustrates a bar graph comparing the proportion of DME patients having a BCVA improvement of greater than or equal to 20 letters in patient groups receiving example embodiment bioerodible implants according to example embodiment methods disclosed herein.
Figure 3 illustrates a line graph comparing the mean change in BCVA between different DME patient groups receiving example embodiment bioerodible implants according to example embodiment methods disclosed herein.
Figure 4 illustrates a bar graph comparing the mean average decrease from baseline of CSRT in DME patient groups receiving example embodiment bioerodible implants according to example embodiment methods disclosed herein.
Figure 5 illustrates a bar graph comparing the proportion of DME patients having a BCVA improvement of greater than or equal to 15 letters in patient groups receiving example embodiment bioerodible implants according to example embodiment methods disclosed herein.
Figure 6 shows a table listing common adverse events occurring during a study according to the examples.
Figure 7 shows a table listing ocular surgeries performed to correct IOP during a study according to the examples.

### DETAILED DESCRIPTION

### Definitions

The following terms as used herein have the following meanings:
"Active agent" and "drug" are used interchangeably and refer to any substance used to treat an ocular condition.
"Bioerodible polymer" means a polymer which degrades *in vivo*, and wherein erosion of the polymer over time is required to achieve the active agent release kinetics according to the present invention. Thus, hydrogels such as methylcellulose which act to release drug through polymer swelling are specifically excluded from the term "bioerodible (or biodegradable) polymer". The words "bioerodible" and "biodegradable" are synonymous and are used interchangeably herein.
"Injury" or "damage" are interchangeable and refer to the cellular and morphological manifestations and symptoms resulting from an inflammatory-mediated condition, such as, for example, inflammation.
"Ocular condition" means a disease, aliment or condition which affects or involves the eye or one or the parts or regions of the eye, such as a retinal disease. The eye includes the eyeball and the tissues and fluids which constitute the eyeball, the periocular muscles (such as the oblique and rectus muscles) and the portion of the optic nerve which is within or adjacent to the eyeball. :"Ocular condition" is synonymous with "medical condition of the eye"
"Plurality" means two or more.
"Posterior ocular condition" means a disease, ailment or condition which affects or involves a posterior ocular region or site such as choroid or sclera (in a position posterior to a plane through the posterior wall of the lens capsule), vitreous, vitreous chamber, retina, optic nerve (i.e. the optic disc), and blood vessels and nerve which vascularize or innervate a posterior ocular region or site.
"Steroidal anti-inflammatory agent" and "glucocorticoid" are used interchangeably herein, and are meant to include steroidal agents, compounds or drugs which reduce inflammation when administered at a therapeutically effective level.
"Suitable for insertion (or implantation) in (or into) an ocular region or site" with regard to an implant, means an implant which has a size (dimensions) such that it can be inserted or implanted without causing excessive tissue damage and without unduly physically interfering with the existing vision of the patient into which the implant is implanted or inserted.
"Therapeutic levels" or "therapeutic amount" means an amount or a concentration of an active agent that has been locally delivered to an ocular region that is appropriate to safely treat an ocular condition so as to reduce or prevent a symptom of an ocular condition.

According to some embodiments, a bioerodible implant for use in treating a medical condition of the eye comprises an active agent dispersed within a biodegradable polymer matrix. Example bioerodible implants and methods of making such implants are described in U.S. Patent No. 8,034,370, U.S. Patent No. 8,242,099, U.S. Patent No. 7,767,223, and U.S. Patent No. 8,257,730. The active agent is dexamethasone.

The bioerodible implant is sized for implantation in the vitreous of the human. The ocular region can be any one or more of the anterior chamber, the posterior chamber, the vitreous cavity, the choroid, the suprachoroidal space, the conjunctiva, the subconjunctival space, the episcleral space, the intracorneal space, the epicorneal space, the sclera, the pars plana, surgically-induced avascular regions, the macula, and the retina.

A method for making a bioerodible implant for use in treating a medical condition of the eye can include a plurality of extrusions of a biodegradable polymer. This method can also comprise the step of milling the biodegradable polymer prior to the extrusion. The biodegradable polymer is a poly(lactic-co-glycolic)acid (PLGA) copolymer. The ratio of lactic to glycolic acid monomers in the polymer can be about 50/50 weight percentage. Additionally, the PLGA copolymer can be about 20 to about 90 weight percent of the bioerodible implant. Alternately, the PLGA copolymer can be about 40 percent by weight of the bioerodible implant.

Disclosed herein are biodegradable ocular implants and such implants for use in methods for treating medical conditions of the eye. Usually, the implants are formed to be monolithic, i.e., the particles of active agent are distributed throughout the biodegradable polymer matrix. Furthermore, the implants are formed to release an active agent into an ocular region of the eye over various time periods. The active agent can be released over a time period including, but is not limited to, approximately twelve months, ten months, nine months, eight months, six months, seven months, eight months, three months, one month, or less than one month.

### Biodegradable Implants For Use In Treating Medical Conditions of the Eye

The implants include an active agent dispersed within a biodegradable polymer. In some embodiments, dexamethasone is the only active agent present in the implant.

The steroidal anti-inflammatory agent, dexamethasone, can constitute from about 10% to about 90% by weight of the implant. In one variation, the agent is from about 40% to about 80% by weight of the implant. In a preferred variation, the agent comprises about 60% by weight of the implant.

### The Biodegradable Polymer Matrix

In one variation, the active agent is homogeneously dispersed in the biodegradable polymer matrix of the implants. The selection of the biodegradable polymer matrix to be employed will vary with the desired release kinetics, patient tolerance, the nature of the disease to be treated, and the like. Polymer characteristics that are considered include, but are not limited to, the biocompatibility and biodegradability at the site of implantation, compatibility with the active agent of interest, and processing temperatures. The biodegradable polymer matrix usually comprises at least about 10, at least about 20, at least about 30, at least about 40, at least about 50, at least about 60, at least about 70, at least about 80, or at least about 90 weight percent of the implant. In one variation, the biodegradable polymer matrix comprises about 40% by weight of the implant.

Biodegradable polymer matrices which may be employed include, but are not limited to, polymers made of monomers such as organic esters or ethers, which when degraded result in physiologically acceptable degradation products. Anhydrides, amides, orthoesters, or the like, by themselves or in combination with other monomers, may also be used. The polymers are generally condensation polymers. The polymers may be crosslinked or non-crosslinked. If crosslinked, they are usually not more than lightly crosslinked, and are less than 5% crosslinked, usually less than 1% crosslinked.

Of particular interest are polymers of hydroxyaliphatic carboxylic acids, either homo- or copolymers, and polysaccharides. Included among the polyesters of interest are homo- or copolymers of D-lactic acid, L-lactic acid, racemic lactic acid, glycolic acid, caprolactone, and combinations thereof. Copolymers of glycolic and lactic acid are of particular interest, where the rate of biodegradation is controlled by the ratio of glycolic to lactic acid. The percent of each monomer in poly(lactic-co-glycolic)acid (PLGA) copolymer may be 0-100%, about 15-85%, about 25-75%, or about 35-65%. In a preferred variation, a 50/50 PLGA copolymer is used . More preferably, a random copolymer of 50/50 PLGA is used.

Biodegradable polymer matrices that include mixtures of hydrophilic and hydrophobic ended PLGA are employed, and are useful in modulating polymer matrix degradation rates. Hydrophobic ended (also referred to as capped or end-capped) PLGA has an ester linkage hydrophobic in nature at the polymer terminus. Typical hydrophobic end groups include, but are not limited to alkyl esters and aromatic esters. Hydrophilic ended (also referred to as uncapped) PLGA has an end group hydrophilic in nature at the polymer terminus. Examples of suitable hydrophilic end groups that may be incorporated to enhance hydrolysis include, but are not limited to, carboxyl, hydroxyl, and polyethylene glycol. The specific end group will typically result from the initiator employed in the polymerization process. For example, if the initiator is water or carboxylic acid, the resulting end groups will be carboxyl and hydroxyl. Similarly, if the initiator is a monofunctional alcohol, the resulting end groups will be ester or hydroxyl.

In general, the ratio of hydrophilic end to hydrophobic end PLGA in the biodegradable polymer matrices useful according to this invention range from about 10:1 to about 1:10 by weight. For example, the ratio may be 3:1, 2:1, or 1:1 by weight. In a preferred variation, an implant with a ratio of hydrophilic end to hydrophobic end PLGA of 3:1 w/w is used.

### Additional Agents

Other agents may be employed in the formulation for a variety of purposes. For example, buffering agents and preservatives may be employed. Preservatives which may be used include, but are not limited to, sodium bisulfite, sodium bisulfate, sodium thiosulfate, benzalkonium chloride, chlorobutanol, thimerosal, phenylmercuric acetate, phenylmercuric nitrate, methylparaben, polyvinyl alcohol and phenylethyl alcohol. Examples of buffering agents that may be employed include, but are not limited to, sodium carbonate, sodium borate, sodium phosphate, sodium acetate, sodium bicarbonate, and the like, as approved by the FDA for the desired route of administration. Electrolytes such as sodium chloride and potassium chloride may also be included in the formulation.

The biodegradable ocular implants may also include additional hydrophilic or hydrophobic compounds that accelerate or retard release of the active agent. Furthermore, the inventors believe that because hydrophilic end PLGA has a higher degradation rate than hydrophobic end PLGA due to its ability to take up water more readily, increasing the amount of hydrophilic end PLGA in the implant polymer matrix will result in faster dissolution rates. The time from implantation to significant release of active agent (lag time) increases with decreasing amounts of hydrophilic end PLGA in the ocular implant. The lag time for implants having 0% hydrophilic end PLGA (40% w/w hydrophobic end) was shown to be about 21 days. In comparison, a significant reduction in lag time was seen with implants having 10% w/w and 20% w/w hydrophilic end PLGA.

### Applications

The medical condition of the eye which is treated by the implant is diabetic macular edema.

### Method of Implantation

The biodegradable implants may be inserted into the eye by a variety of methods, including placement by forceps, by trocar, or by other types of applicators, after making an incision in the sclera. In some instances, a trocar or applicator may be used without creating an incision. In a variation, a hand held applicator is used to insert one or more biodegradable implants into the eye. The hand held applicator typically comprises an 18-30 GA stainless steel needle, a lever, an actuator, and a plunger.

The method of implantation generally first involves accessing the target area within the ocular region with the needle. Once within the target area, e.g., the vitreous cavity, the lever on the hand held device is depressed to cause the actuator to drive the plunger forward. As the plunger moves forward, it pushes the implant into the target area.

According to an embodiment, the long axis of an applicator having a needle having a bevel can be held parallel to the limbus, and the sclera can be engaged at an oblique angle with the bevel of the needle upwards (away from the sclera) to create a shelved scleral path. The tip of the needle can then be advanced within the sclera for about 1 mm (parallel to the limbus), then re-directed toward the center or the eye and advanced until penetration of the sclera is completed and the vitreous cavity of a patient's eye is entered. After that, the applicator can be activated to deliver a biodegradable implant within the vitreous of the patient.

### Bioerodible Implant For Use In A Method Of Treatment Of DME

In an embodiment, a bioerodible implant for use in a method of treating diabetic macular edema is provided, the method comprising injecting said bioerodible implant into the vitreous of a patient in need thereof, at a frequency of once every 7 months, 8 months or 9 months, the bioerodible implant comprising dexamethasone homogeneously dispersed within a biodegradable polymer matrix. The bioerodible implant is of the types disclosed herein. According to some embodiments, the administration of the implant to the eye of the patient includes injecting a bioerodible implant into the vitreous.

The methods are used to treat diabetic macular edema.

To "treat," as used here, means to deal with medically. It includes, for example, administering the bioerodible implant useful according to the invention to prevent the onset of DME as well as to alleviate its severity.

The implant is administered once every 7 months, 8 months or 9 months. In some embodiments, the bioerodible implant is administered at one or more of the frequencies described above for the remainder of the lifetime of the patient. The bioerodible implant is administered at one or more of the frequencies described above for a period of time of 2 years, 3 years, 4 years, 5 years, 10 years, 15 years, for the lifetime of the patient, or until the ocular condition (such as DME) is adequately treated. Such methods at such dosing regimens described above can be therapeutically effective to treat DME in a patient in need thereof. In some embodiments, the methods described herein can increase the visual acuity of a patient having DME

Since the implant is biodegradable, subsequent implant(s) can be inserted without the need for surgical removal of the existing implant. By avoiding the peak vitreous drug concentrations produced by the need for frequent repeat injections, the implant may potentially reduce the risk of unwanted side effects, such as cataract formation, IOP elevation, and glaucoma and it may reduce the risk of injection-related complications, such as lens injury, retinal detachment, and infectious endophthalmitis.

According to some embodiments, the bioerodible implant can treat an ocular condition in a patient having diabetic macular edema independent of the lens status of the patient. For example, in some embodiments, the treatment of DME can be achieved using the implants and methods disclosed herein regardless of whether a patient has a phakic lens or a pseudophakic lens. According to some embodiments, treatment of DME can be achieved in patients having a pseudophakic lens. According to some embodiments, treatment of DME can be achieved in patients having a phakic lens, but who are scheduled for or plan to have cataract surgery.

According to some embodiments, the bioerodible implant can treat an ocular condition in a patient who is refractory to other existing treatments for DME For example, according to some methods, a patient having DME who is refractive to anti-VEGF intraocular injections can effectively be treated by the methods disclosed herein. According to some other methods, a patient having DME who is refractive to laser photocoagulation can effectively be treated by the methods disclosed herein.

### EXAMPLES

The following examples are provided for the purposes of further describing the embodiments described herein.

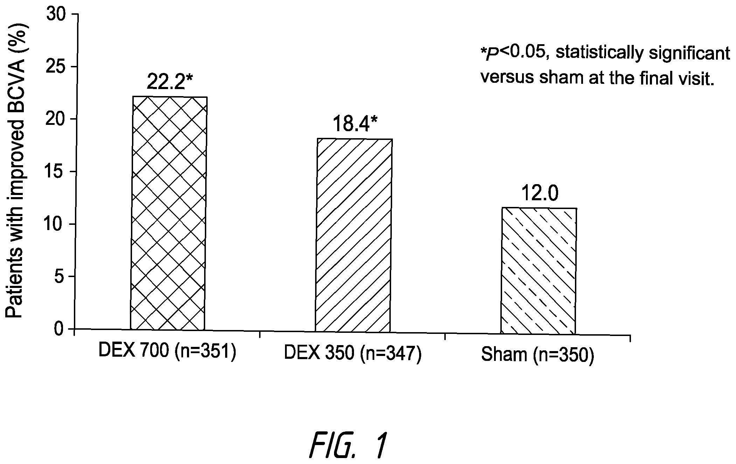

A Phase III, multicenter, masked, randomized, sham-controlled trial was conducted to assess the safety and efficacy of 700 µg and 350 µg dexamethasone posterior segment drug delivery system in patients with diabetic macular edema ("DME"). The study was conducted over a period of three years. The results of these studies are shown in Figures 1-7.

The implants used in the study were comprised of dexamethasone and a polymer matrix of 50:50 poly (D,L-lactide-co-glycolide) PLGA, constituted of two grades of PLGA (50:50 PLGA ester and 50:50 PLGA acid). See Table 1 for details. The two PLGAs combination as presented in Table 2 was chosen for the biodegradable polymer matrix. General properties of the chosen PLGAs are presented in Table 3.

**Table 1: Qualitative composition of a sample DEX PS DDS**

| **Component** | **Quality Standard** | **Function** |
|---|---|---|
| Dexamethasone | Ph. Eur. | Active ingredient |
| 50:50 PLGA ester | Allergan, Inc. | Biodegradable extended release polymer matrix |
| 50:50 PLGA acid | Allergan, Inc. | Biodegradable extended release polymer matrix |

**Table 2: Quantitative Composition of a sample DEX PS DDS (manufacturing batch formula)**

| **Component** | | | **350 µg** | **700 µg** |
|---|---|---|---|---|
| | | | | |
| Dexamethasone | | | 350 µg (60%) | 700 µg (60%) |
| 50:50 PLGA ester (hydrophobic) | | | 58 µg (10%) | 116 µg (10%) |
| 50:50 PLGA acid (hydrophilic) | | | 175 µg (30%) | 350 µg (30%) |

**Table 3 General properties of PLGAs**

| | **50:50 PLGA ester** | **50:50 PLGA acid** |
|---|---|---|
| Common Names | Resomer RG 502, PLG, PLGA, Poly (lactic-glycolic) acid, 50:50 Poly (D,L-lactide-co-glycolide), Polylactic/Polyglycolic acid, Polyglactin 910 | Resomer RG 502H, PLG acid end, PLGA acid end, 50:50 Poly (D,L-lactide-co-glycolide) acid end |
| Structure | 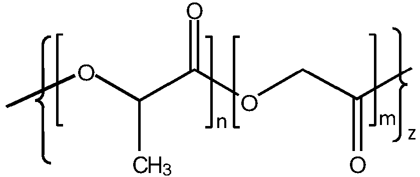 Where: | 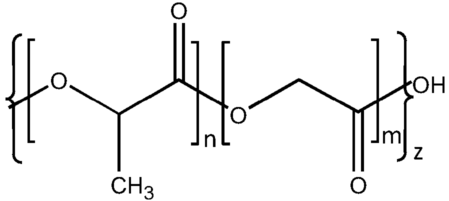 Where: |
| | n=m | n = m |
| | n = number of lactide repeating units | n = number of lactide repeating units |
| | m = number of glycolide repeating units | m = number of glycolide repeating units |
| | z = overall number of lactide-co-glycolide repeating units | z = overall number of lactide-co-glycolide repeating units |
| CAS Number | 34346-01-5 | 26780-50-7 |
| Empirical Formula | [(C3H4O2)x . (C2H2O2)y]CH3, x:y=50:50 | [(C3H4O2)x . (C2H2O2)y]OH, x:y=50:50 |
| Description | white to off white powder | white to near white powder |

In the trial, patients having DME received either a bioerodible implant having 700 µg of dexamethasone, a bioerodible implant having 350 µg of dexamethasone, or a bioerodible implant having 0 µg of dexamethasone (a sham). The implants were injected into the vitreous of one eye of each patient. The patients were evaluated for retreatment every 3 months after a month 6 visit. Retreatment (i.e. administration of another implant) was allowed every 6 months. Retreatment was allowed if central retinal thickness in the patient was greater than 175 µm or if there was any evidence of residual retinal edema. Patients were allowed a maximum of 7 implants over the three-year study period per eye.

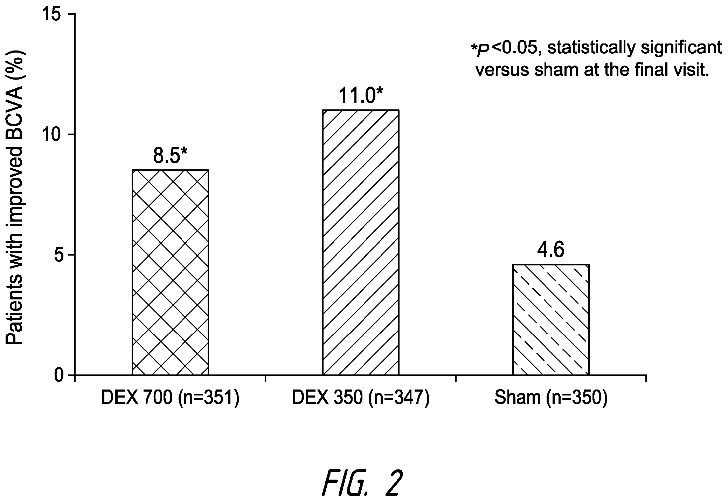

As illustrated in Figures 1-2, a statistically significant improvement in Best Corrected Visual Acuity ("BCVA") score was achieved in patients receiving the 700 µg dexamethasone implant and in patients receiving the 350 µg dexamethasone implant compared to patients receiving the sham implant. Figure 1 illustrates that 22.2% of patients receiving the 700 µg dexamethasone implant demonstrated a BCVA improvement greater than or equal to 15 letters and that 18.4% of patients receiving the 350 µg dexamethasone implant demonstrated a BCVA improvement greater than or equal to 15 letters. Figure 2 illustrates that 8.5% of patients receiving the 700 µg dexamethasone implant demonstrated a BCVA improvement greater than or equal to 20 letters and that 11.0% of patients receiving the 350 µg dexamethasone implant demonstrated a BCVA improvement greater than or equal to 20 letters.

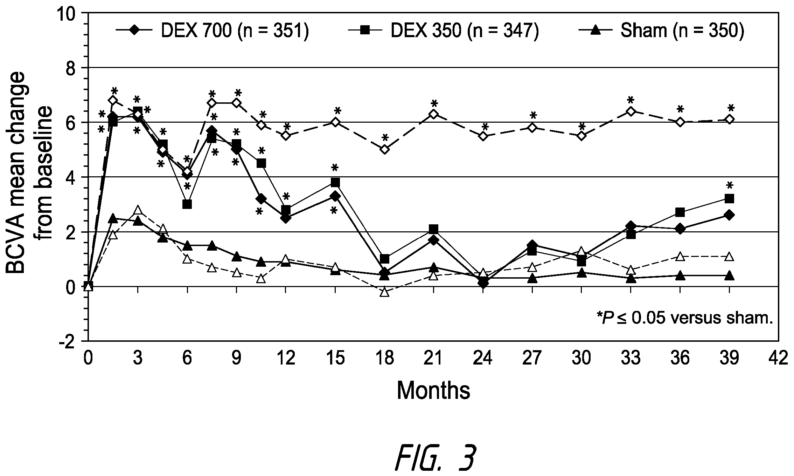

Figure 3 illustrates that patients in groups receiving the 700 µg and 350 µg dexamethasone-containing implants generally showed a greater improvement in BCVA over the three-year study period than the patients receiving the sham implant. As shown in Figure 3, a rapid increase in BCVA was observed in patients receiving the dexamethasone implants. Specifically, a mean BCVA increase of about 6 letters from baseline was observed over the first about 3 months of treatment for patients receiving the 350 µg dexamethasone implant, and a mean BCVA increase of about 7 letters from baseline was observed over the first about 3 months of treatment for patients receiving the 700 µg dexamethasone implant.

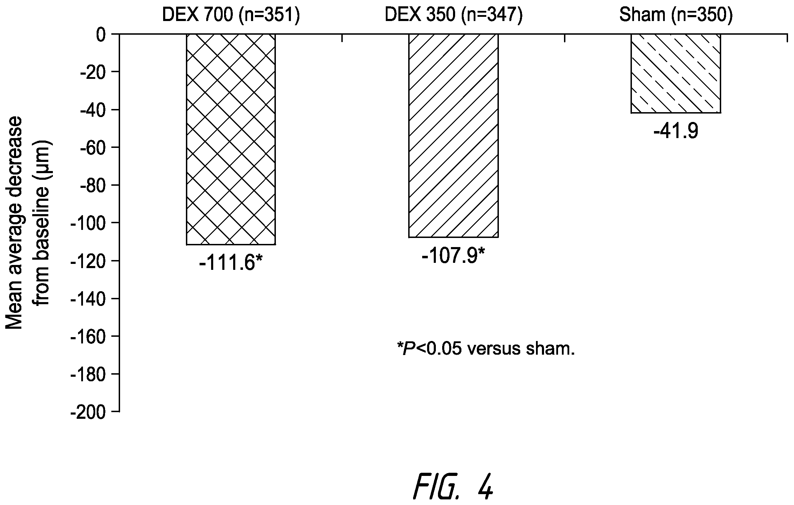

As illustrated in Figure 4, the mean average decrease from baseline in Central Subfield Retinal Thickness ("CSRT") was greater in patients receiving the dexamethasone implants than in patients receiving the sham implant. Figure 4 illustrates that patients receiving the 700 µg dexamethasone implant demonstrated a CSRT mean average decrease of 111.6 µm from baseline and that patients receiving the 350 µg dexamethasone implant demonstrated a CSRT mean average decrease of 107.9 µm from baseline.

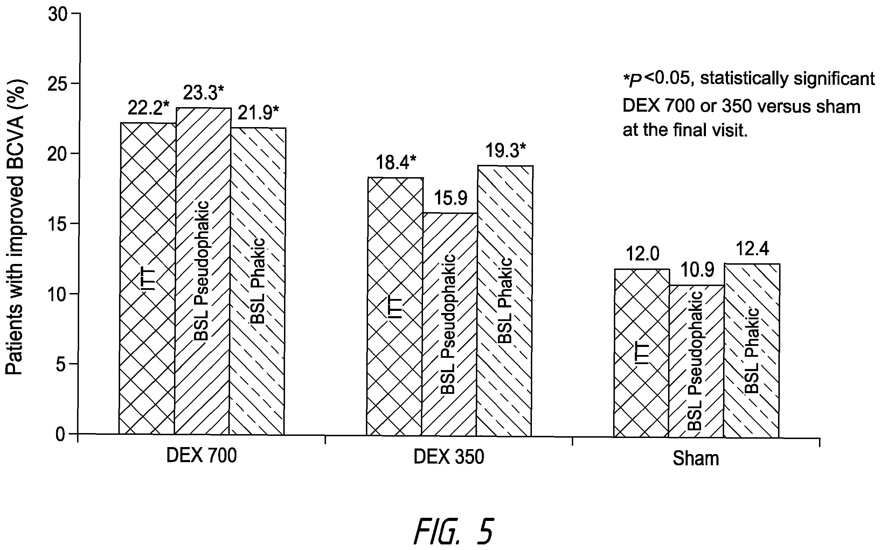

As illustrated in Figure 5, the dexamethasone implants led to significant BCVA improvements, regardless of the lens status of the patient at baseline.

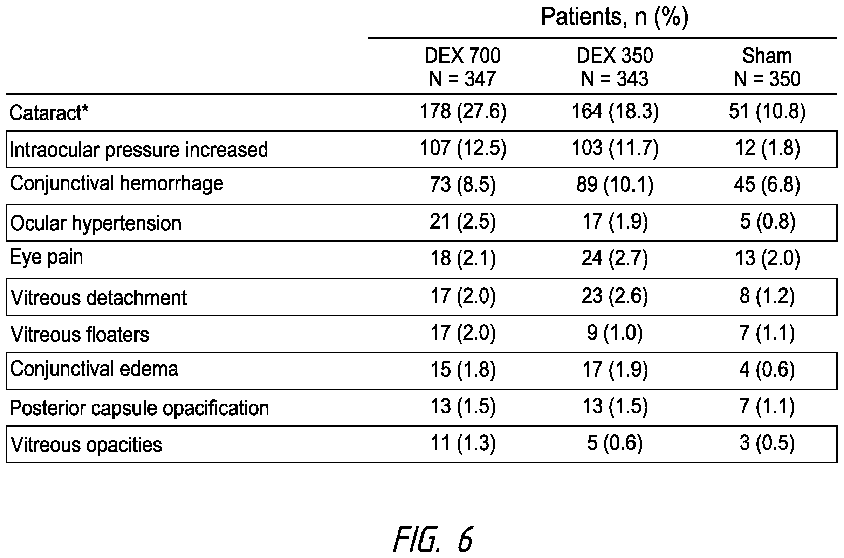

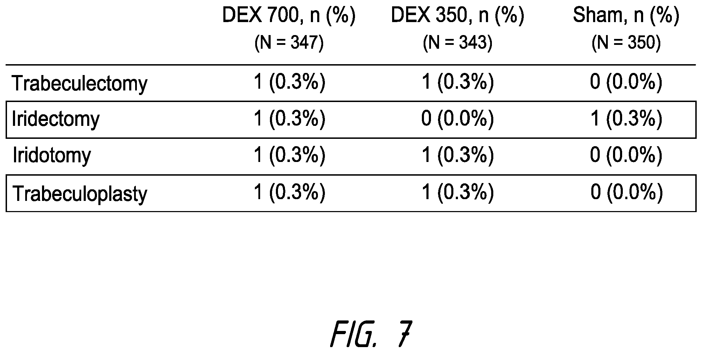

The adverse event profile from the study is shown below in Figure 6. As shown in the Figure, the most common adverse events experienced in the study were cataracts and intraocular pressure. However, despite the occurrence of the adverse event of increased IOP, surprisingly, very few patients (about 0.3% per surgery type per study group) underwent surgery during the study for management of IOP. The number of patients who underwent surgery and the type of surgeries underwent are illustrated in Figure 7.

As shown from the data in the Figures, the implants and methods disclosed herein resulted in significant, long term improvement in vision in patients with diabetic macular edema. The proportion of patients with a greater than or equal to 15-letter gain was significantly higher with the 350 µg and 700 µg dexamethasone implants compared with sham at Year 3. The treatment benefit was observed with a mean of 4.1 injections over 3 years.

Table 4 below illustrates the visual acuity outcomes at Month 39 of the study.

**Table 4**

| **Study** | **Outcomes** | **OZURDEX^{®}** | **Sham** | **Estimated Difference (95% CI)** |
|---|---|---|---|---|
| 1^{a} | Mean (SD) Baseline BCVA (Letters) | 56 (10) | 57 (9) | |
| | Median (range) Baseline BCVA (Letters) | 59 (34-95) | 58 (34-74) | |
| | Gain of ≥ 15 letters in BCVA (n(%)) | 34 (21%) | 19 (12%) | 9.3% (1.4%, 17.3%) |
| | Loss of ≥ 15 letters in BCVA (n(%)) | 15 (9%) | 17 (10%) | -1.1% (-7.5%, 5.3%) |
| | Mean change in BCVA (SD) | 4.1 (13.9) | 0.9 (11.9) | 3.2 (0.4, 5.9) |
| 2^{b} | Mean (SD) Baseline BCVA (Letters) | 55 (10) | 56 (9) | |
| | Median (range) Baseline BCVA (Letters) | 58 (34-72) | 58 (36-82) | |
| | Gain of ≥ 15 letters in BCVA (n(%)) | 30 (18%) | 16(10%) | 8.4% (0.9%, 15.8%) |
| | Loss of ≥ 15 letters in BCVA (n(%)) | 30 (18%) | 18 (11%) | 7.1% (-0.5%, 14.7%) |
| | Mean change in BCVA (SD) | 0.4 (17.5) | 0.8 (13.6) | -0.7 (-4.1, 2.6) |
| ^{a}Study 1: **OZURDEX**^{®}, N=163: Sham, N=165 | | | | |
| ^{b}study 2: **OZURDEX**^{®}, N=165: Sham, N=163 | | | | |
| ^{c}14% (16.8% from **OZURDEX**^{®} and 12.2% from Sham) of patients had BCVA outcome at Month 39, for the remaining patients, the data at Month 36 or earlier was carried forward. | | | | |

Table 5 below illustrates the best corrected visual acuity outcomes for the pseudophakic and phakic subgroups.

**Table 5**

| **Subgroup (Pooled)** | **Outcomes** | **OZURDEX^{®}** | **Sham** | **Estimated Difference (95% CI)** |
|---|---|---|---|---|
| ^{a}Pseudophakic | Gain of ≥ 15 letters in BCVA (n(%)) | 16(20%) | 11 (11%) | 8.4% (-2.2%, 19.0%) |
| | Loss of ≥ 15 letters in BCVA (n(%)) | 4 (5%) | 7 (7%) | -2.2% (-9.1%, 4.7%) |
| | Mean change in BCVA (SD) | 5.8 (11.6) | 1.4 (12.3) | 4.2 (0.8, 7.6) |
| ^{b}Phakic | Gain of ≥ 15 letters in BCVA (n(%)) | 48 (20%) | 24 (11%) | 9.0% (2.7%, 15.4%) |
| | Loss of ≥ 15 letters in BCVA (n(%)) | 41 (17%) | 28 (12%) | 4.4% (-1.9%, 10.7%) |
| | Mean change in BCVA (SD) | 1.0 (16.9) | 0.6 (12.9) | 0.3 (-2.4,3.0) |
| ^{a} Pseudophakic: **OZURDEX^{®}**, N=82; Sham, N=99 | | | | |
| ^{b} Phakic: **OZURDEX^{®}**, N=246; Sham. N=229 | | | | |
| ^{c}14% (16.8% from **OZURDEX^{®}** and 12.2% from Sham) of patients had BCVA outcome at Month 39, for the remaining patients the data at Month 36 or earlier was used in the analysis. | | | | |

## Claims

1. A bioerodible implant for use in a method for treating diabetic macular edema (DME), the method comprising injecting said bioerodible implant into the vitreous of a human at a frequency of once every 7 months, 8 months or 9 months, the bioerodible implant comprising a continuous, double extruded rod comprising dexamethasone homogeneously dispersed within a biodegradable polymer matrix; wherein
the biodegradable polymer matrix comprises a mixture of poly(D,L-lactide-co-glycolide) (PLGA) having hydrophilic end groups and poly(D,L-lactide-co-glycolide) (PLGA) having hydrophobic end groups;
wherein the bioerodible implant is sized for implantation in the vitreous of the human; and wherein the method is therapeutically effective to treat DME; and
wherein said bioerodible implant is administered for a period of time of 2 years, 3 years, 4 years, 5 years, 10 years, 15 years, or for the lifetime of said human.

2. The bioerodible implant for use of Claim 1 wherein the dexamethasone is present in the bioerodible implant in an amount of 60% by weight, based on the total weight of the bioerodible implant.

3. The bioerodible implant for use of Claim 1 or Claim 2, wherein the PLGA having hydrophobic end groups is present in the bioerodible implant in an amount of 10% by weight, based on the total weight of the bioerodible implant.

4. The bioerodible implant for use of any one of Claims 1-3, wherein the PLGA having hydrophilic end groups is present in the bioerodible implant in an amount of 30% by weight, based on the total weight of the bioerodible implant.

5. The bioerodible implant for use of any one of Claims 1-4, wherein the human has a pseudophakic lens.

6. The bioerodible implant for use of any one of Claims 1-4, wherein the human has a phakic lens.

7. The bioerodible implant for use of any one of Claims 1-6, wherein the method results in an increase of visual acuity in the human.

## Patentansprüche

1. Bioerodierbares Implantat zur Verwendung in einem Verfahren zur Behandlung von diabetischem Makulaödem (DMÖ), wobei das Verfahren die Injektion des bioerodierbaren Implantats in den Glaskörper eines Menschen in einer Häufigkeit von einmal alle 7 Monate, 8 Monate oder 9 Monate umfasst, wobei das bioerodierbare Implantat ein kontinuierliches, doppelt extrudiertes Stäbchen umfasst, das Dexamethason, das homogen in einer biologisch abbaubaren Polymermatrix dispergiert ist, umfasst;
wobei die biologisch abbaubare Polymermatrix eine Mischung aus Poly(D,L,-lactid-co-glycolid) (PLGA), das hydrophile Endgruppen aufweist, und Poly(D,L-lactid-co-glycolid) (PLGA), das hydrophobe Endgruppen aufweist, umfasst;
wobei das bioerodierbare Implantat für eine Implantation in den Glaskörper des Menschen bemessen ist; und worin das Verfahren zur Behandlung von DMÖ therapeutisch wirksam ist; und
wobei das bioerodierbare Implantat für einen Zeitraum von 2 Jahren, 3 Jahren, 4 Jahren, 5 Jahren, 10 Jahren, 15 Jahren oder für die Lebensdauer des Menschen verabreicht wird.

2. Bioerodierbares Implantat zur Verwendung gemäß Anspruch 1, wobei das Dexamethason im bioerodierbaren Implantat in einer Menge von 60 Gew.-%, bezogen auf das Gesamtgewicht des bioerodierbaren Implantats, vorhanden ist.

3. Bioerodierbares Implantat zur Verwendung gemäß Anspruch 1 oder 2, wobei das PLGA, das hydrophobe Endgruppen aufweist, im bioerodierbaren Implantat in einer Menge von 10 Gew.-%, bezogen auf das Gesamtgewicht des bioerodierbaren Implantats, vorhanden ist.

4. Bioerodierbares Implantat zur Verwendung gemäß mindestens einem der Ansprüche 1-3, wobei das PLGA, das hydrophile Endgruppen aufweist, im bioerodierbaren Implantat in einer Menge von 30 Gew.-%, bezogen auf das Gesamtgewicht des bioerodierbaren Implantats, vorhanden ist.

5. Bioerodierbares Implantat zur Verwendung gemäß mindestens einem der Ansprüche 1-4, wobei der Mensch eine pseudophakische Linse aufweist.

6. Bioerodierbares Implantat zur Verwendung gemäß mindestens einem der Ansprüche 1-4, wobei der Mensch eine phakische Linse aufweist.

7. Bioerodierbares Implantat zur Verwendung gemäß mindestens einem der Ansprüche 1-6, wobei das Verfahren zu einer Erhöhung der Sehschärfe beim Menschen führt.

## Revendications

1. Implant bioérodable destiné à être utilisé dans un procédé de traitement de l'œdème maculaire diabétique (OMD), le procédé comprenant l'injection dudit implant bioérodable dans le vitré d'un être humain à une fréquence d'une fois tous les 7 mois, 8 mois ou 9 mois, l'implant bioérodable comprenant une tige continue à double extrusion comprenant de la dexaméthasone dispersée de manière homogène dans une matrice polymère biodégradable ; dans lequel
la matrice polymère biodégradable comprend un mélange de poly(D,L-lactide-co-glycolide) (PLGA) ayant des groupes terminaux hydrophiles et de poly(D,L-lactide-co-glycolide) (PLGA) ayant des groupes terminaux hydrophobes ;
dans lequel l'implant bioérodable est dimensionné pour être implanté dans le vitré de l'être humain ; et dans lequel le procédé est thérapeutiquement efficace pour traiter l'OMD ; et
dans lequel ledit implant bioérodable est administré pendant une période de 2 ans, 3 ans, 4 ans, 5 ans, 10 ans, 15 ans ou pendant toute la durée de vie dudit être humain.

2. L'implant bioérodable destiné à être utilisé selon la revendication 1, dans lequel la dexaméthasone est présente dans l'implant bioérodable en une quantité de 60 % en poids, par rapport au poids total de l'implant bioérodable.

3. L'implant bioérodable destiné à être utilisé selon la revendication 1 ou la revendication 2, dans lequel le PLGA ayant des groupes terminaux hydrophobes est présent dans l'implant bioérodable en une quantité de 10 % en poids, par rapport au poids total de l'implant bioérodable.

4. L'implant bioérodable destiné à être utilisé selon l'une quelconque des revendications 1 à 3, dans lequel le PLGA ayant des groupes terminaux hydrophiles est présent dans l'implant bioérodable en une quantité de 30 % en poids, par rapport au poids total de l'implant bioérodable.

5. L'implant bioérodable destiné à être utilisé selon l'une quelconque des revendications 1 à 4, dans lequel l'être humain a une lentille pseudophaque.

6. L'implant bioérodable destiné à être utilisé selon l'une quelconque des revendications 1 à 4, dans lequel l'être humain possède un cristallin phaque.

7. L'implant bioérodable destiné à être utilisé selon l'une quelconque des revendications 1 à 6, dans lequel le procédé entraîne une augmentation de l'acuité visuelle chez l'être humain.
